# EUROPÄISCHE PATENTSCHRIFT

(11) **EP 0 837 107 B1**
(45) Veröffentlichungstag und Bekanntmachung des Hinweises auf die Patenterteilung: **12.09.2001**
(21) Anmeldenummer: 97117678.9
(22) Anmeldetag: 13.10.1997
(51) Int. Cl.: C09B 19/02, C07D 498/22

(54) **Verfahren zur Herstellung von Dioxazin-Verbindungen**
Process for the manufacture of dioxazine compounds
Procédé de fabrication de composés de dioxazine

(30) Priorität: 21.10.1996 DE 19643344
(43) Veröffentlichungstag der Anmeldung: 22.04.1998
(73) Patentinhaber: Clariant GmbH, 65929 Frankfurt am Main (DE)
(72) Erfinder: Nagl, Gert, Dr., 61338 Niederdorfelden (DE); Bauer, Wolfgang, Dr., 63477 Maintal (DE); Urban, Manfred, 65205 Wiesbaden (DE); Schnaitmann, Dieter, Dr., 65817 Eppstein (DE)

(56) Entgegenhaltungen:
- EP-A- 0 036 966
- FR-A- 2 587 705
- YOSHIFUMI MAKI ET AL: "photo-oxidative cyclisation of 2'-hydroxychalcones leading to flavones induced by heterocycle N-oxides" TETRAHEDRON, Bd. 44, Nr. 11, 1988, GB, Seiten 3187-3194, XP002053097
- DATABASE WPI Section Ch, Week 8739 Derwent Publications Ltd., London, GB; Class E23, AN 87-274731 XP002053098 & JP 62 192 385 A (SUMITOMO CHEM IND KK) , 22.August 1987
- DATABASE WPI Section Ch, Week 8150 Derwent Publications Ltd., London, GB; Class E23, AN 81-92192D XP002053099 & JP 56 141 355 A (SUMITOMO CHEM CO LTD) , 5.November 1981
- DATABASE WPI Section Ch, Week 9313 Derwent Publications Ltd., London, GB; Class E23, AN 93-104467 XP002053100 & JP 05 043 808 A (NIPPON KAYAKU KK) , 23.Februar 1993

## Beschreibung

Die vorliegende Erfindung betrifft ein verbessertes Verfahren zur Herstellung von Dioxazin-Verbindungen der allgemeinen Formel I,

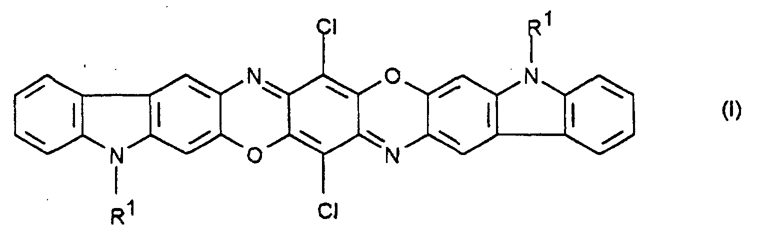

Diese Dioxazin-Verbindungen werden zur Herstellung wertvoller Farbstoffe und Pigmente verwendet (s. Ullmann's Encyclopedia of Industrial Chemistry, Vol. A 3, p. 233) und können technisch beispielsweise durch Ringschluß von Verbindungen der allgemeinen Formel II 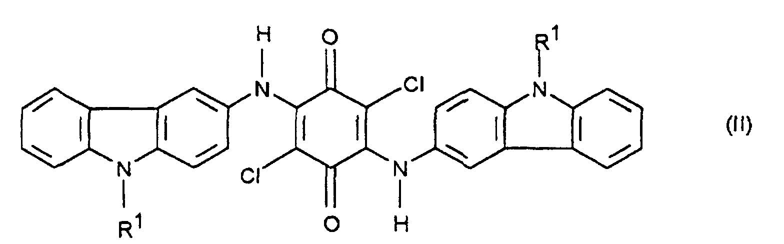 mit Benzolsulfonsäurechlorid oder 4-Toluolsulfonsäurechlorid als Ringschlußmittel in hergestellt werden (siehe DE-A 30 10 949).
Als Ringschlußmittel wurden außerdem Benzolsulfonsäurechloride vorgeschlagen, die am Benzolring elektronenanziehende Substituenten tragen (siehe JP Hei 7-145327). Es ist auch bereits bekannt, zur Verbesserung der Ausbeute den Ringschluß in Gegenwart eines Phasentransfer-Katalysators (siehe JP Sho 56-141355) oder einer organischen Base (siehe JP Hei 6-179682) durchzuführen. Auch der Ringschluß unter vermindertem Druck wurde bereits beschrieben (siehe JP Hei 5-43808). Als Ausbeute werden in den genannten Dokumenten bestenfalls 87% der Theorie erreicht (siehe Beispiele 1 und 3 der JPHei 5-43808).
Nachteilig ist bei allen Methoden, bei denen als Ringschlußmittel Benzolsulfonsäurechlorid oder dessen Derivate eingesetzt werden, die Tatsache, daß aus dem Ringschlußmittel eine große Menge an nicht verwertbarem Abfall entsteht. Die Abfallmenge wird außerdem dadurch vermehrt, daß die erzielbare Ausbeute noch weit von der Theorie entfernt ist Der daraus resultierende Verbesserungsbedarf im Hinblick auf Schonung der Resourcen und Entlastung der Umwelt wurde bereits erkannt.
So werden in der JP Sho 62-192385 zu diesem Zweck als Ringschlußmittel heterocyclische Amine vorgeschlagen, die zurückgewonnen werden können. Dadurch entfällt zwar ein Teil des Abfalls. Die so hergestellten Produkte enthielten jedoch wesentlich weniger Chlor als sich nach der allgemeinen Formel (I) errechnet. Außerdem ließ sich die Ausbeute nicht auf erforderliche Werte steigern.

Es stellt sich somit die Aufgabe, ein Ringschluß-Verfahren für die Herstellung der Dioxazin-Verbindungen der allgemeinen Formel (I) bereitzustellen, das folgende Kriterien erfüllt:
Der Ringschluß soll mit einem Ringschlußmittel bewirkt werden, das keinen Abfall erzeugt.
Das Produkt soll in einer sehr gut filtrierenden Form anfallen und im Finish ein Pigment mit hoher Farbstärke, reinem rotstichigem Farbton und sehr guten Echtheitseigenschaften ergeben.

Es wurde überraschenderweise gefunden, daß sich die Dioxazin-Verbindungen der allgemeinen Formel (1) unter Erfüllung der genannten Kriterien herstellen lassen, wenn als Ringschlußmittel anstelle von Benzolsulfonsäurechlorid oder Toluolsulfonsäurechlorid ein N-Hetaren-N-oxid verwendet wird.

Die vorliegende Erfindung betrifft somit ein Verfahren zur Herstellung von Dioxazin-Derivaten der allgemeinen Formel I 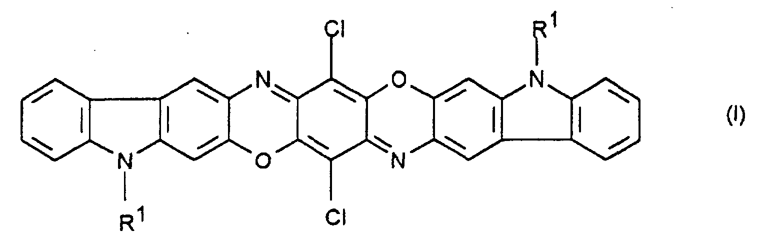 worin R¹ Wasserstoff oder (C₁-C₈)-Alkyl bedeutet, durch Ringschluß einer Verbindung der allgemeinen Formel II 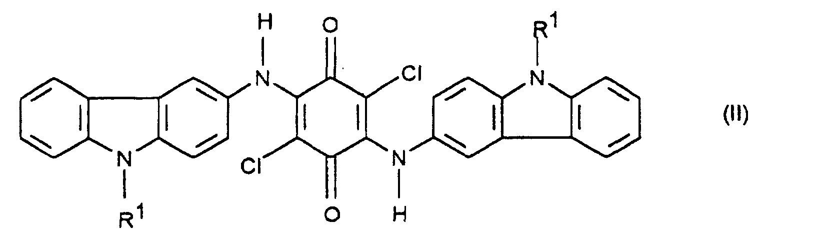 in Gegenwart eines Ringschlußmittels, dadurch gekennzeichnet, daß als Ringschlußmittel ein N-Hetaren-N-oxid eingesetzt wird.

Für R¹ stehendes (C₁-C₈)-Alkyl kann geradkettig oder verzweigt sein und beispielseise für Methyl, Ethyl, n-Propyl, i-Propyl, n-Butyl, sek.Butyl, i-Butyl, tert.Butyl, Pentyl, Hexyl, Heptyl oder Octyl stehen. Bevorzugt ist Ethyl.

Geeignete Hetaren-N-oxide sind beispielsweise Verbindungen der allgemeinen Formel III 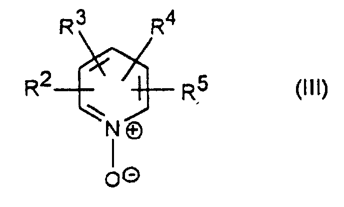 worin R², R³, R⁴ und R⁵ unabhängig voneinander Wasserstoff, (C₁-C₄)-Alkyl, Chlor oder Cyan bedeuten, Verbindungen der allgemeinen Formel IV oder IVa 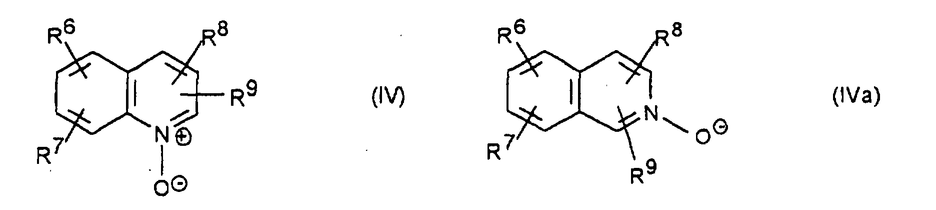 worin R⁶, R⁷, R⁸ und R⁹ unabhängig voneinander Wasserstoff oder (C₁-C₄)-Alkyl bedeuten, sowie Verbindungen der allgemeinen Formel V 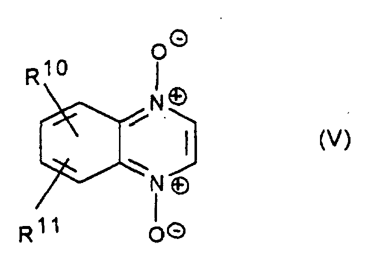 worin R¹⁰ und R¹¹ unabhängig voneinander Wasserstoff oder (C₁-C₄)-Alkyl bedeuten.

Die für R² bis R¹¹ stehenden (C₁-C₄)-Alkylreste können geradkettig oder verzweigt sein und beispielsweise Methyl, Ethyl, n-Propyl, i-Propyl, n-Butyl, sek-Butyl, i-Butyl oder tert.-Butyl sein.

Bevorzugte Hetaren-N-oxide sind insbesondere solche, die billig und technisch leicht herstellbar sind. Beispiele sind Pyridin-N-oxid und die N-Oxide der verschiedenen Picoline. Pyridin-N-oxid ist besonders bevorzugt. Die N-Hetaren-N-oxide werden bei der Ringschluß-Reaktion zu N-Hetaren und Wasser reduziert. Wenn das entstehende N-Hetaren unterhalb der Reaktionstemperatur siedet, kann es aus dem Reaktionsgemisch während der Reaktion abdestilliert und anschließend nach bekannten Verfahren wieder zu dem N-Hetaren-N-oxid aufoxidiert werden, z.B. nach US 3826746, 4504666, FR-A 2587705 und EP-A 224662.
Die N-Hetaren-N-oxide können in reiner Form oder im Gemisch oder als Lösungen in inerten organischen Lösungsmitteln oder in Wasser eingesetzt werden. Die Menge des zugegebenen N-Hetaren-N-oxids kann in weiten Grenzen variiert werden. Bevorzugt wird das N-Hetaren-N-oxid in einer Menge von 1 bis 3 mol, bezogen auf 1 mol eingesetzte Verbindung der allgemeinen Formel II, zugegeben. Besonders bevorzugt sind Mengen von 1,2 bis 2,5 mol, bezogen auf 1 mol eingesetzte Verbindung der allgemeinen Formel II.

Es wurde gefunden, daß die Ausbeute an Verbindung der allgemeinen Formel I nahe an die Theorie gesteigert werden kann, wenn das erfindungsgemäße Verfahren in Gegenwart eines Chinons ausgeführt wird. In einer bevorzugten Ausführungsform des erfindungsgemäßen Verfahrens wird deshalb die Ringschlußreaktion in Gegenwart einer Chinon-Verbindung ausgeführt.
Als Chinon-Verbindung können alle Chinone eingesetzt werden, die in der Literatur als Dehydrierungsmittel beschrieben sind. Bevorzugt sind billige und technisch leicht herstellbare Chinone. Besonders bevorzugt ist p-Benzochinon, ganz besonders bevorzugt Chloranil.
Die Menge des zugesetzten Chinons kann in weiten Grenzen variiert werden. Größere Mengen schaden der Reaktion zwar nicht. Um jedoch die Abfallmenge sehr niedrig zu halten, setzt man relativ wenig zu. Bevorzugt wird das Chinon in einer Menge von 5 bis 50 Molprozent, bezogen auf die Verbindung der allgemeinen Formel II, eingesetzt. Besonders bevorzugt ist eine Chinon-Menge von 10 bis 40 Molprozent.

Die erfindungsgemäße Ringschlußreaktion erfolgt bevorzugt bei Temperaturen zwischen 150°C und 200°C. Als Reaktionsmedium sind inerte organische Lösungsmittel mit entsprechend hoher Siedetemperatur geeignet. Geeignet sind beispielsweise Alkylbenzole, Dialkylbenzole, Alkylnaphthaline, Alkane, Alkene, o-Dichlorbenzol, m-Dichlorbenzol oder p-Dichlorbenzol. Bevorzugtes Reaktionsmedium ist o-Dichlorbenzol. Die Reaktion kann auch unter reduziertem Druck erfolgen. Die Reaktionsgeschwindigkeit ist vergleichbar mit dem konventionellen Ringschluß mit Benzolsulfonsäurechloriden. Beispielsweise ist in siedendem o-Dichlorbenzol der Ringschluß spätestens nach drei Stunden praktisch beendet.

In der Regel wird am Ende der Reaktionszeit das Reaktionsgemisch heiß filtriert und die erhaltene Paste mit heißem Lösungsmittel gewaschen und getrocknet. Um möglicherweise in geringen Mengen entstandene wasserlösliche Nebenprodukte zu entfernen, wird das Produkt in der Regel noch mit Wasser extrahiert.

Die Verbindungen der allgemeinen Formel II können beispielsweise in an sich bekannter Weise durch Kondensation von 3-Amino-N-alkyl-carbazolen der allgemeinen Formel VI 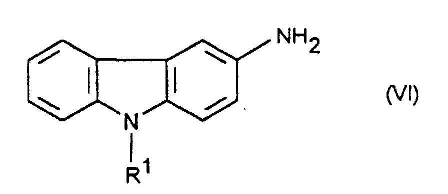 worin R¹ wie oben angegeben definiert ist mit Chloranil in einem inerten organischen Lösungsmittel hergestellt werden. Als inerte organische Lösungsmittel können dabei beispielsweise Alkylbenzole, Dialkylbenzole, Alkylnaphthaline, Alkane oder Alkene verwendet werden (siehe beispielsweise JP Hei-7-331097 und JP Hei-7-331098).
Nach diesem Verfahren erhaltene Verbindung der allgemeinen Formel II kann in Form der dabei erhaltenen Suspension direkt, das heißt ohne Zwischenisolierung, der erfindungsgemäßen Ringschlußreaktion unterworfen werden. Wurde bei der Kondensation mit einem Chloranil-Überschuß gearbeitet, braucht der Ringschlußreaktion kein Chinon mehr zugesetzt werden.

Die vorliegende Erfindung betrifft auch ein Verfahren zur Herstellung von Dioxazin-Derivaten der allgemeinen Formel I, 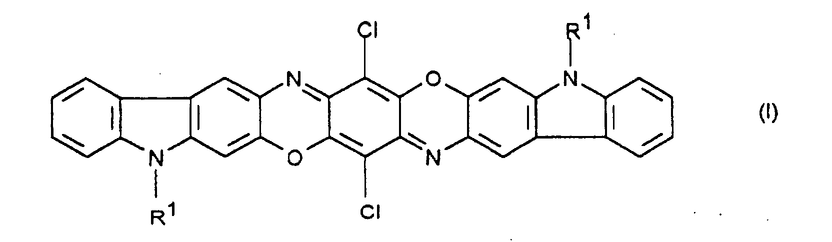 worin R¹ Wasserstoff oder (C₁-C₈)-Alkyl bedeutet durch Kondensation eines 3-Amino-N-alkyl-carbazols der allgemeinen Formel VI 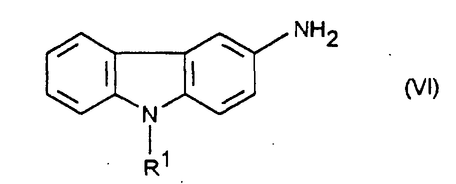 worin R¹ wie oben angegeben definiert ist mit Chloranil in einem inerten organischen Lösungsmittel zu einem Kondensationsprodukt der allgemeinen Formel II 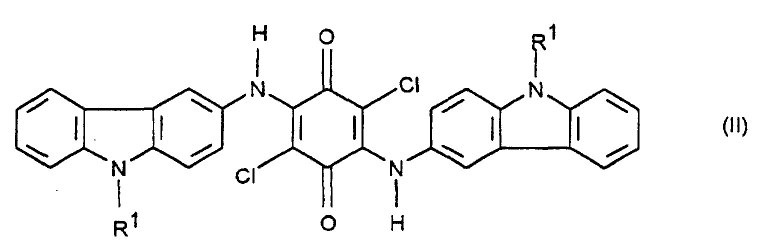 und anschließendem Ringschluß zur Verbindung der allgemeinen Formel I in Gegenwart eines Ringschlußmittels, dadurch gekennzeichnet, daß als Ringschlußmittel ein N-Hetaren-N-oxid eingesetzt wird.

Als inerte organische Lösungsmittel können beispielsweise Alkylbenzole, Dialkylbenzole, Alkylnaphthaline, Alkane und Alkene eingesetzt werden. Bevorzugt ist o-Dichlorbenzol.

Pro Mol Verbindung der allgemeinen Formel VI wird Chloranil bevorzugt in Mengen von 0,50 bis 0,75 Mol, besonders bevorzugt 0,55 bis 0,65 Mol eingesetzt.

Die nachfolgenden Beispiele dienen der Erläuterung des erfindungsgemäßen Verfahrens.

### Beispiel 1:

In einem 500 ml - Vierhalskolben mit KPG-Glasflügelrührer, Tropftrichter, Thermometer, Destillierbrücke und Ölbad werden 41,6 g 2,5-Dichlor-3,6-bis(9-ethyl-3-carbazolylamino)-1,4-benzochinon in 390 g o-Dichlorbenzol angeschlämmt. Man erhitzt das Gemisch auf Siedetemperatur. Anschließend wird eine Lösung von 15 g Pyridin-N-oxid in 15 g o-Dichlorbenzol unter Rühren und Abdestillieren in einer halben Stunde zugetropft. Danach wird drei Stunden lang unter weiterem Abdestillieren nachgerührt. Insgesamt destillieren etwa 100 ml ab. Das Reaktionsgemisch wird heiß filtriert. Die Paste wird mit 500 g heißem o-Dichlorbenzol gewaschen und bei 100 °C im Vakuum getrocknet. Die getrocknete Masse wird in 500 g Wasser bei ca.60 °C verrührt, filtriert und mit Wasser gewaschen. Nach dem Trocknen erhält man 33,6 g der Dioxazin-Verbindung der Formel I, in der R¹ eine Ethyl-Gruppe ist (81,3 % d. Theorie). Daß es sich um diese Verbindung handelt, wurde durch IR-Absorptionsspektren, Röntgenbeugung, Ausprüfung als Pigment und Elementaranalyse bestätigt.

### Beispiel 2:

In einem 500 ml - Vierhalskolben mit KPG-Glasflügelrührer, Tropftrichter, Thermometer, Destillierbrücke und Ölbad werden 41,6 g 2,5-Dichlor-3,6-bis(9-ethyl-3-carbazolylamino)-1,4-benzochinon in 390 g o-Dichlorbenzol angeschlämmt. Man gibt 5,7 g Chloranil zu und erhitzt das Gemisch auf Siedetemperatur. Unter Rühren und Abdestillieren wird eine Lösung von 12,0 g Pyridin-N-oxid in 12 g o-Dichlorbenzol in etwa 15 Minuten zugetropft. Anschließend wird drei Stunden lang unter weiterem Abdestillieren nachgerührt. Insgesamt destillieren etwa 100 ml ab. Das Reaktionsgemisch wird heiß filtriert. Die Paste wird mit 500 g heißem o-Dichlorbenzol gewaschen und bei 100 °C im Vakuum getrocknet. Die getrocknete Masse wird in 500 g Wasser bei ca.60 °C verrührt, filtriert und mit Wasser gewaschen. Nach dem Trocknen erhält man 40,7 g der Dioxazin-Verbindung der Formel I, in der R¹ eine Ethyl-Gruppe ist (98,5 % d. Theorie). Daß es sich um diese Verbindung handelt, wurde durch IR-Absorptionsspektren, Röntgenbeugung, Ausprüfung als Pigment und Elementaranalyse bestätigt.

### Beispiele 3 - 7:

Es wurde wie in dem Beispiel 2 gearbeitet mit dem Unterschied, daß anstelle von Pyridin-N-oxid und/oder anstelle von Chloranil andere N-Hetaren-N-oxide bzw. andere Chinon-Verbindungen eingesetzt wurden.
Die Ausbeute an der Dioxazin-Verbindung der Formel I, in der R¹ eine Ethyl-Gruppe ist, zeigt die Tabelle 1:

**Tabelle 1**

| Beispiel | N-Hetaren-N-oxid (Menge) | Chinon (Menge) | Ausbeute in g | Ausbeute (%der Theorie) |
|---|---|---|---|---|
| 3 | Pyridin-N-oxid (14 g) | p-Benzochinon (2 g) | 35,0 | 84,7 |
| 4 | Pyridin-N-oxid (14 g) | 2,3-Dichlor-1,4-naphthochinon (4 g) | 34,5 | 83,5 |
| 5 | 2-Picolin-N-oxid (14g) | Chloranil (5,5 g) | 39,4 | 95,3 |
| 6 | 4-Picolin-N-oxid (15 g) | Chloranil (4 g) | 37,7 | 91,2 |
| 7 | 4-Picolin-N-oxid (15 g) | p-Benzochinon (2 g) | 39,1 | 94,6 |

### Vergleichsbeispiel 1 (konventionelles Verfahren):

In einem 500 ml -Vierhalskolben mit KPG-Glasflügelrührer, Thermometer, Destillierbrücke und Ölbad werden 41,6 g 2,5-Dichlor-3,6-bis(9-ethyl-3-carbazolylamino)-1,4-benzochinon in 390 g o-Dichlorbenzol angeschlämmt. Man gibt 17 g Benzolsulfonsäurechlorid zu und erhitzt das Gemisch auf Siedetemperatur. Unter Abdestillieren wird drei Stunden lang nachgerührt. Insgesamt destillieren etwa 100 ml ab. Das Reaktionsgemisch wird heiß filtriert. Die Paste wird mit 500 g heißem o-Dichlorbenzol gewaschen und bei 100°C im Vakuum getrocknet. Die getrocknete Masse wird in 500 g Wasser bei ca.60°C verrührt, filtriert und mit Wasser gewaschen. Nach dem Trocknen erhält man 34,7 g der Dioxazin-Verbindung der Formel I, in der R¹ eine Ethyl-Gruppe ist (84,0% d. Theorie).

### Beispiel 8:

In einen 1000 ml-Vierhalskolben mit KPG-Glasflügelrührer, Tropftrichter, Thermometer, Destillationsvorrichtung und Ölbad gibt man 840 g einer 7,5%-igen technischen Lösung von 3-Amino-9-ethylcarbazol in o-Dichlorbenzol. Nach Zudosieren von 39 g Chloranil und 16 g Soda wird bei 30-65°C nachgerührt, bis nach DC kein 3-Amino-9-ethyl-carbazol mehr nachzuweisen ist. Man destilliert das Reaktionswasser unter Erhitzen auf 160°C ab und erhitzt weiter auf Siedetemperatur. Anschließend wird eine Lösung von 32 g Pyridin-N-oxid in 32 g o-Dichlorbenzol unter Rühren und Abdestillieren in einer halben Stunde zugetropft. Danach wird drei Stunden lang unter weiterem Abdestillieren nachgerührt. Insgesamt destillieren etwa 250 ml ab. Das Reaktionsgemisch wird heiß filtriert. Die Paste wird mit 1000 g heißem o-Dichlorbenzol gewaschen und bei 100°C im Vakuum getrocknet. Die getrocknete Masse wird in 1000 g Wasser bei ca. 60°C verrührt, filtriert und mit Wasser gewaschen. Nach dem Trocknen erhält man 73,3 g der Dioxazin-Verbindung der Formel I, in der R¹ eine Ethyl-Gruppe ist (82,9 % d. Theorie). Daß es sich um diese Verbindung handelt, wurde durch IR-Absorptionsspektren, Röntgenbeugung. Ausprüfung als Pigment und Elementaranalyse bestätigt.

### Beispiel 9:

In einen 1000 ml - Vierhalskolben mit KPG-Glasflügelrührer, Tropftrichter, Thermometer, Destillationsvorrichtung und Ölbad gibt man 840 g einer 7,5%-igen technischen Lösung von 3-Amino-9-ethylcarbazol in o-Dichlorbenzol. Nach Zudosieren von 39 g Chloranil und 16 g Soda wird bei 30-65°C nachgerührt, bis nach DC kein 3-Amino-9-ethyl-carbazol mehr nachzuweisen ist. Man destilliert das Reaktionswasser unter Erhitzen auf 160°C ab, setzt dem Gemisch anschließend 4,5 g Chloranil zu und erhitzt auf Siedetemperatur. Anschließend wird eine Lösung von 25 g Pyridin-N-oxid in 25 g o-Dichlorbenzol unter Rühren und Abdestillieren in einer halben Stunde zugetropft. Danach wird drei Stunden lang unter weiterem Abdestillieren nachgerührt. Insgesamt destillieren etwa 250 ml ab. Das Reaktionsgemisch wird heiß filtriert. Die Paste wird mit 1000 g heißem o-Dichlorbenzol gewaschen und bei 100°C im Vakuum getrocknet. Die getrocknete Masse wird in 1000 g Wasser bei ca.60°C verrührt, filtriert und mit Wasser gewaschen. Nach dem Trocknen erhält man 79,1 g der Dioxazin-Verbindung der Formel I, in der R¹ eine Ethyl-Gruppe ist (89,4 % d. Theorie). Daß es sich um diese Verbindung handelt, wurde durch IR-Absorptionsspektren, Röntgenbeugung, Ausprüfung als Pigment und Elementaranalyse bestätigt.

### Beispiel 10:

Es wird wie in Beispiel 9 gearbeitet mit dem einzigen Unterschied, daß dem Gemisch 9 g Chloranil zugesetzt werden. Man erhält 86,1 g der Dioxazin-Verbindung der Formel I, in der R¹ eine Ethyl-Gruppe ist (97,4 % d. Theorie). Daß es sich um diese Verbindung handelt, wurde durch IR-Absorptionsspektren, Röntgenbeugung, Ausprüfung als Pigment und Elementaranalyse bestätigt.

### Beispiel 11:

In einen 1000 ml - Vierhalskolben mit KPG-Glasflügelrührer, Tropftrichter, Thermometer, Destillationsvorrichtung und Ölbad gibt man 840 g einer 7,5%-igen technischen Lösung von 3-Amino-9-ethylcarbazol in o-Dichlorbenzol. Nach Zudosieren von 39 g Chloranil und 16 g Soda wird bei 30-65°C nachgerührt, bis nach DC kein 3-Amino-9-ethyl-carbazol mehr nachzuweisen ist. Man destilliert das Reaktionswasser unter Erhitzen auf 160°C ab und gibt anschließend 9 g Chloranil und 25 g Pyridin-N-oxid zu. Das Gemisch wird weiter auf Siedetemperatur erhitzt. In einer Stunde werden 210 ml abdestilliert. Anschließend werden in 2 Stunden 210 ml o-Dichlorbenzol zugetropft und gleichzeitig 210 ml abdestilliert. Danach wird heiß filtriert. Die Paste wird mit 1000 g heißem o-Dichlorbenzol gewaschen und bei 100°C im Vakuum getrocknet. Die getrocknete Masse wird in 1000 g Wasser bei ca.60°C verrührt, filtriert und mit Wasser gewaschen. Nach dem Trocknen erhält man 84,5 g der Dioxazin-Verbindung der Formel I, in der R¹ eine Ethyl-Gruppe ist (95,5 % d. Theorie). Daß es sich um diese Verbindung handelt, wurde durch IR-Absorptionsspektren, Röntgenbeugung, Ausprüfung als Pigment und Elementaranalyse bestätigt.

### Beispiel 12:

In einen 1000 ml - Vierhalskolben mit KPG-Glasflügelrührer, Tropftrichter, Thermometer, Destillationsvorrichtung und Ölbad gibt man 840 g einer 7,5%-igen technischen Lösung von 3-Amino-9-ethylcarbazol in o-Dichlorbenzol. Nach Zudosieren von 39 g Chloranil und 16 g Soda wird bei 30-65°C nachgerührt, bis nach DC kein 3-Amino-9-ethyl-carbazol mehr nachzuweisen ist. Man destilliert das Reaktionswasser unter Erhitzen auf 160°C ab, setzt dem Gemisch anschließend 4,5 g p-Benzochinon zu und erhitzt auf Siedetemperatur. Anschließend wird eine Lösung von 35 g 4-Picolin-N-oxid in 35 g o-Dichlorbenzol unter Rühren und Abdestillieren in einer halben Stunde zugetropft. Danach wird drei Stunden lang unter weiterem Abdestillieren nachgerührt. Insgesamt destillieren etwa 250 ml ab. Das Reaktionsgemisch wird heiß filtriert. Die Paste wird mit 1000 g heißem o-Dichlorbenzol gewaschen und bei 100°C im Vakuum getrocknet. Die getrocknete Masse wird in 1000 g Wasser bei ca.60°C verrührt, filtriert und mit Wasser gewaschen. Nach dem Trocknen erhält man 84,7 g der Dioxazin-Verbindung der Formel I, in der R¹ eine Ethyl-Gruppe ist (95,8 % d. Theorie). Daß es sich um diese Verbindung handelt, wurde durch IR-Absorptionsspektren, Röntgenbeugung, Ausprüfung als Pigment und Elementaranalyse bestätigt.

### Vergleichsbeispiel 2 (konventionelles Verfahren):

In einen 1000 ml - Vierhalskolben mit KPG-Glasflügelrührer, Thermometer, Destillationsvorrichtung und Ölbad gibt man 840 g einer 7,5%-igen technischen Lösung von 3-Amino-9-ethylcarbazol in o-Dichlorbenzol. Nach Zudosieren von 39 g Chloranil und 16 g Soda wird bei 30-65°C nachgerührt, bis nach DC kein 3-Amino-9-ethyl-carbazol mehr nachzuweisen ist. Man destilliert das Reaktionswasser unter Erhitzen auf 160 °C ab und gibt anschließend 35 g Benzolsulfonsäurechlorid zu. Das Gemisch wird weiter auf Siedetemperatur erhitzt und 3 Stunden unter Abdestillieren nachgerührt. Anschließend wird heiß filtriert. Die Paste wird mit 1000 g heißem o-Dichlorbenzol gewaschen und bei 100°C im Vakuum getrocknet. Die getrocknete Masse wird in 1000 g Wasser bei ca.60°C verrührt, filtriert und mit Wasser gewaschen. Nach dem Trocknen erhält man 76,3 g der Dioxazin-Verbindung der Formel I, in der R¹ eine Ethyl-Gruppe ist (86,3% d. Theorie).

### Anwendungsbeispiel 1:

In einen Kunststoffbehälter, der mit 1400 Teilen zylindrischer Mahlkörper (Cylpebs®, Durchmesser 12 mm, Hersteller: Groh GmbH, Hof) zu 90 Vol-% gefüllt ist, werden nacheinander 22,5 Teile der Dioxazin-Verbindung der Formel I, in der R¹ eine Ethyl-Gruppe ist, hergestellt gemäß einem der vorstehenden Beispiele, und 7,5 Teile wasserfreies Natriumsulfat eingebracht. Die Mischung wird 4 Stunden lang unter Schütteln auf einer Schwingmühle (Typ Vibratom; Hersteller: Siebtechnik Mühlheim) fein vermahlen. Danach wird das Mahlgut von den Mahlkörpern abgesiebt. Man erhält 25,9 Teile Mahlgut.
In einem Rührgefäß werden 37,5 Teile Isobutanol (85 %ig) vorgelegt und nacheinander 2,5 Teile Ameisensäure 98 %ig und 0,38 Teile des Natriumsalzes eines Alkylphenolpolyglykolethersulfats zugegeben. Danach werden 25 Teile des obigen Mahlguts eingetragen und 20 Stunden bei 20-25°C gerührt. Während dieser Zeit werden noch 50 Teile Isobutanol 85 %ig zugesetzt. Anschließend werden 150 Teile Wasser zugegeben, 5 Stunden zum Sieden erhitzt und das Isobutanol durch Erhitzen auf 100 °C am Übergang azeotrop abdestilliert. Nach dem Abkühlen auf 60°C wird das Pigment abgesaugt, mit Wasser neutral und salzfrei gewaschen und bei 80°C getrocknet.
Man erhält 18,6 Teile Pigment, das im Alkydmelaminharz-Lack transparente und farbstarke violette Lackierungen mit roter Nuance liefert. Die Überlackierechtheit ist einwandfrei. Im Nitrocellulosetiefdruck werden transparente und farbstarke Drucke mit hohem Glanz erhalten.

### Anwendungsbeispiel 2:

In einen Kunststoffbehälter, der mit 1400 Teilen zylindrischer Mahlkörper (Cylpebs® , Durchmesser 12 mm, Hersteller: Groh GmbH, Hof) zu 90 Vol-% gefüllt ist, werden 30 Teile der Dioxazin-Verbindung der Formel I, in der R¹ eine Ethyl-Gruppe ist, hergestellt gemäß einem der vorstehenden Beispiele, eingebracht. Das Rohpigment wird 4 Stunden lang unter Schütteln auf einer Schwingmühle (Typ Vibratom; Hersteller: Siebtechnik Mühlheim) fein vermahlen. Danach wird das Mahlgut von den Mahlkörpern abgesiebt. Man erhält 25,9 Teile Mahlgut. In einem Rührgefäß werden 37,5 Teile Isobutanol (85 %ig) vorgelegt und 1,25 Teile Ameisensäure 98 %ig zugegeben. Danach werden 25 Teile des obigen Mahlguts eingetragen und 20 Stunden bei 20-25°C gerührt. Während dieser Zeit werden noch 50 Teile Isobutanol 85 %ig zugesetzt. Anschließend werden 150 Teile Wasser zugegeben und das Isobutanol durch Erhitzen auf 100°C am Übergang azeotrop abdestilliert. Nach dem Abkühlen auf 60°C wird das Pigment abgesaugt, mit Wasser neutral gewaschen und bei 80°C getrocknet.
Man erhält 24,2 Teile Pigment, das in PVC transparente und farbstarke violette Ausfärbungen liefert. Die Ausblutechtheit ist sehr gut.

## Patentansprüche

1. Verfahren zur Herstellung von Dioxazin-Derivaten der allgemeinen Formel I 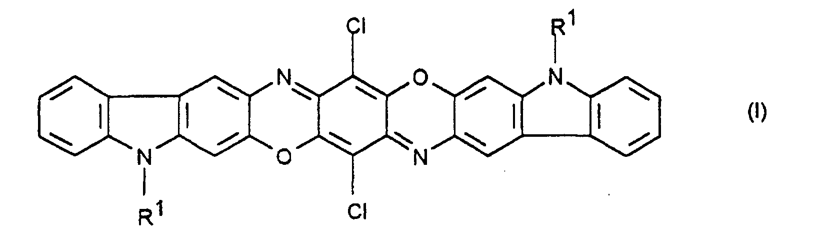 worin R¹ Wasserstoff oder (C₁-C₈)-Alkyl bedeutet durch Ringschluß einer Verbindung der allgemeinen Formel II 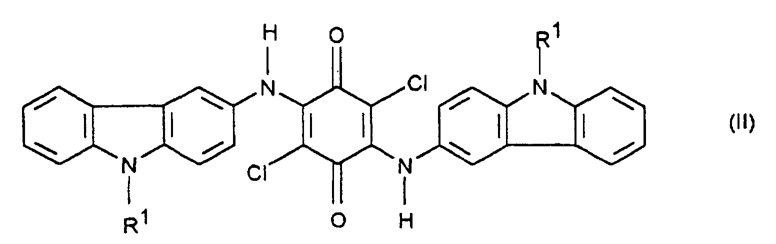 in Gegenwart eines Ringschlußmittels, **dadurch gekennzeichnet, daß** als Ringschlußmittel ein N-Hetaren-N-oxid eingesetzt wird.

2. Verfahren gemäß Anspruch 1, **dadurch gekennzeichnet, daß** R¹ für Ethyl steht.

3. Verfahren gemäß Anspruch 1 und/oder 2, **dadurch gekennzeichnet, daß** als Hetaren-N-oxide Verbindungen der allgemeinen Formel III 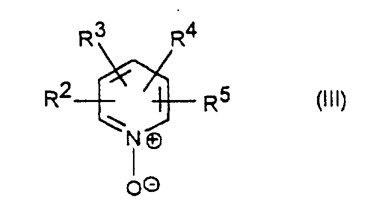 worin R², R³, R⁴ und R⁵ unabhängig voneinander Wasserstoff, (C₁-C₄)-Alkyl, Chlor oder Cyan bedeuten,
Verbindungen der allgemeinen Formel IV oder IVa 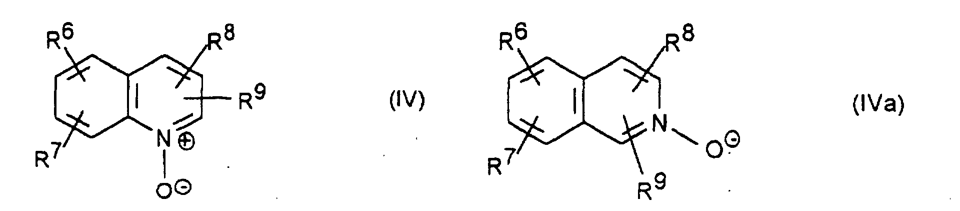 worin R⁶, R⁷, R⁸ und R⁹ unabhängig voneinander Wasserstoff oder (C₁-C₄)-Alkyl bedeuten,
sowie Verbindungen der allgemeinen Formel V 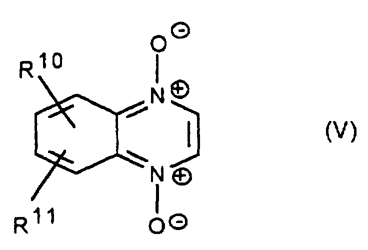 worin R¹⁰ und R¹¹ unabhängig voneinander Wasserstoff oder (C₁-C₄)-Alkyl bedeuten, eingesetzt werden.

4. Verfahren gemäß einem oder mehreren der Ansprüche 1 bis 3, **dadurch gekennzeichnet, daß** als N-Hetaren-N-oxid Pyridin-N-oxid oder ein Picolin-N-oxid eingesetzt wird.

5. Verfahren gemäß einem oder mehreren der Ansprüche 1 bis 4, **dadurch gekennzeichnet, daß** das N-Hetaren-N-oxid in Mengen von 1 bis 3 Mol, besonders bevorzugt in Mengen von 1,2 bis 2,5 Mol, pro Mol Verbindung der allgemeinen Formel II eingesetzt wird.

6. Verfahren gemäß einem oder mehreren der Ansprüche 1 bis 5, **dadurch gekennzeichnet, daß** es in Gegenwart einer Chinon-Verbindung ausgeführt wird.

7. Verfahren gemäß Anspruch 6, **dadurch gekennzeichnet, daß** als Chinon-Verbindung p-Benzochinon oder Chloranil eingesetzt werden.

8. Verfahren gemäß Anspruch 6 und/oder 7, **dadurch gekennzeichnet, daß** die Chinon-Verbindung in Mengen von 5 bis 50 Molprozent, besonders bevorzugt 10 bis 40 Molprozent, bezogen auf die Verbindung der allgemeinen Formel II, eingesetzt wird.

9. Verfahren zur Herstellung von Dioxazin-Derivaten der allgemeinen Formel I 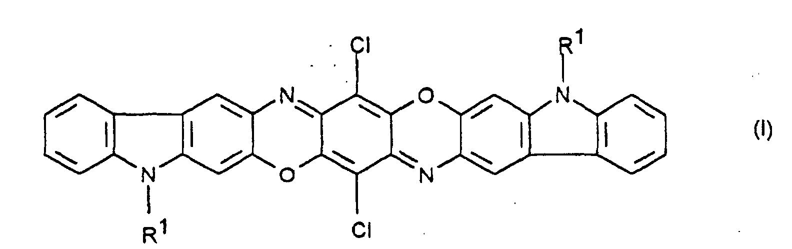 worin R¹ Wasserstoff oder (C₁-C₈)-Alkyl bedeutet durch Kondensation eines 3-Amino-N-alkyl-carbazols der allgemeinen Formel VI 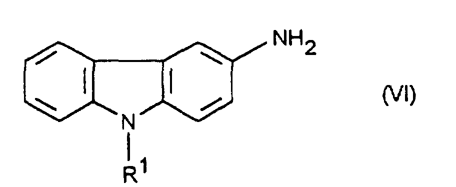 worin R¹ wie oben angegeben definiert ist, mit Chloranil in einem inerten organischen Lösungsmittel zu einem Kondensationsprodukt der allgemeinen Formel II 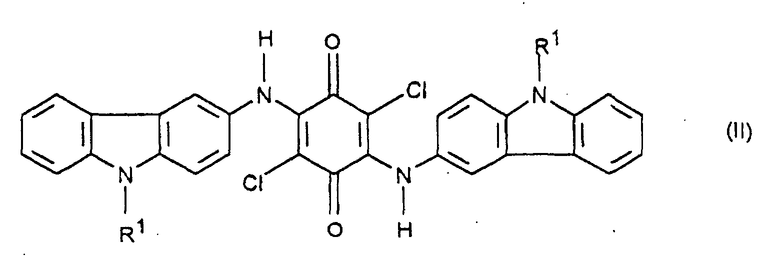 und anschließendem Ringschluß zur Verbindung der allgemeinen Formel I in Gegenwart eines Ringschlußmittels, **dadurch gekennzeichnet, daß** als Ringschlußmittel ein N-Hetaren-N-oxid eingesetzt wird.

10. Verfahren gemäß Anspruch 9, **dadurch gekennzeichnet, daß** die Verbindung der allgemeinen Formel II nicht zwischenisoliert wird.

## Claims

1. A process for the preparation of a dioxazine derivative of the formula I 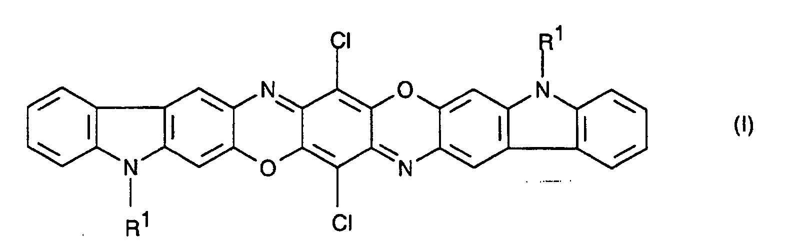 in which R¹ is hydrogen or (C₁-C₈)-alkyl, by ring closure of a compound of the formula II 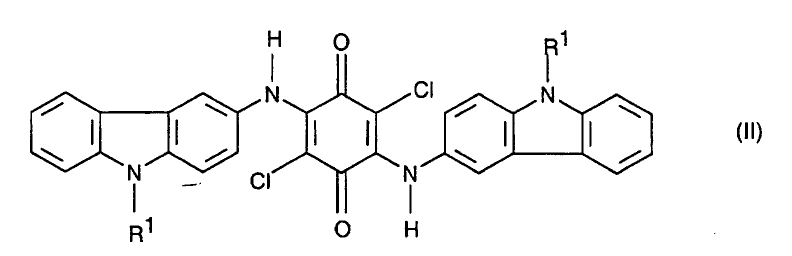 in the presence of a ring-closure agent, wherein the ring-closure agent used is an N-hetarene N-oxide.

2. The process as claimed in claim 1, wherein R¹ is ethyl.

3. The process as claimed in claim 1 and/or 2, wherein the hetarene N-oxides used are compounds of the formula III 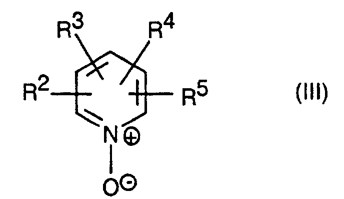 in which R², R³, R⁴ and R⁵, independently of one another, are hydrogen, (C₁-C₄)-alkyl, chlorine or cyano, is a compound of the formula IV or IVa 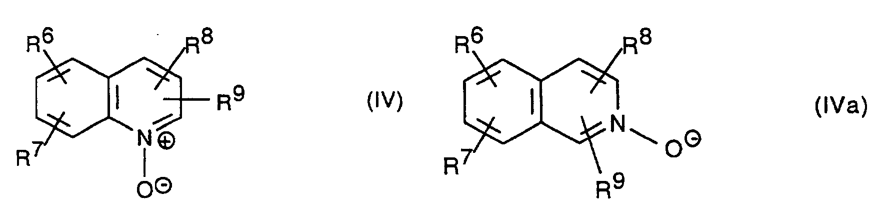 in which R⁶, R⁷, R⁸ and R⁹, independently of one another, are hydrogen or (C₁-C₄)-alkyl, or a compound of the formula V 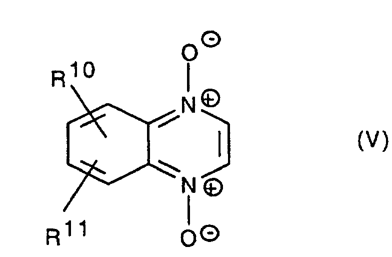 in which R¹⁰ and R¹¹, independently of one another, are hydrogen or (C₁-C₄)-alkyl.

4. The process as claimed in one or more of claims 1 to 3, wherein the N-hetarene N-oxide used is pyridine N-oxide or a picoline N-oxide.

5. The process as claimed in one or more of claims 1 to 4, wherein the N-hetarene N-oxide is used in quantities of from 1 to 3 mol, particularly preferably in quantities of from 1.2 to 2.5 mol, per mole of the compound of the formula II.

6. The process as claimed in one or more of claims 1 to 5, which is carried out in the presence of a quinone compound.

7. The process as claimed in claim 6, wherein the quinone compound used is p-benzoquinone or chloranil.

8. The process as claimed in claim 6 and/or 7, wherein the quinone compound is used in quantities of from 5 to 50 mol percent, particularly preferably from 10 to 40 mol percent, based on the compound of the formula II.

9. The process for the preparation of a dioxazine derivative of the formula I 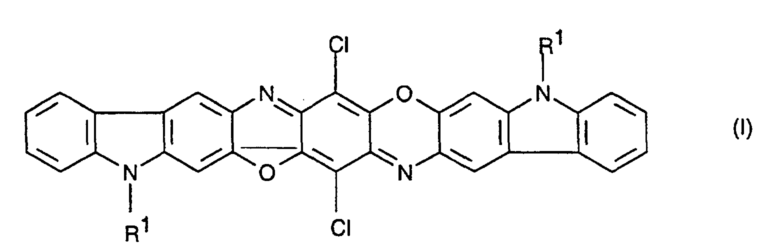 in which R¹ is hydrogen or (C₁-C₈)-alkyl, by condensation of a 3-amino-N-alkylcarbazole of the formula VI 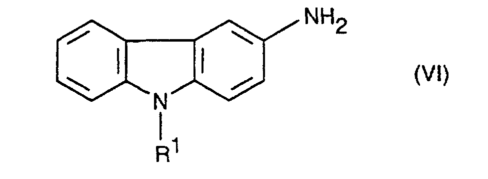 in which R¹ is as defined above, with chloranil in an inert organic solvent to give a condensation product of the formula II 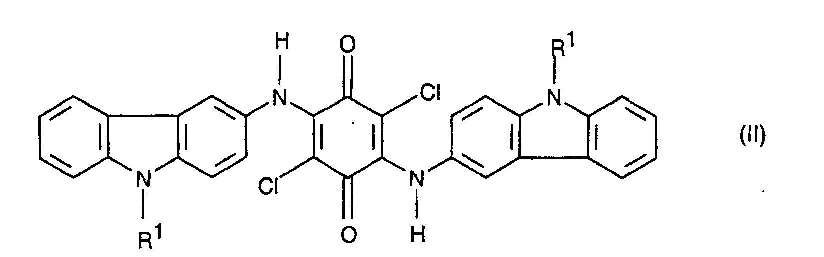 and subsequent ring closure to give a compound of the formula I, in the presence of a ring-closure agent, wherein the ring-closure agent used is an N-hetarene N-oxide.

10. The process as claimed in claim 9, wherein the compound of the formula II is not isolated.

## Revendications

1. Procédé de fabrication de composés de dioxazine de formule générale I 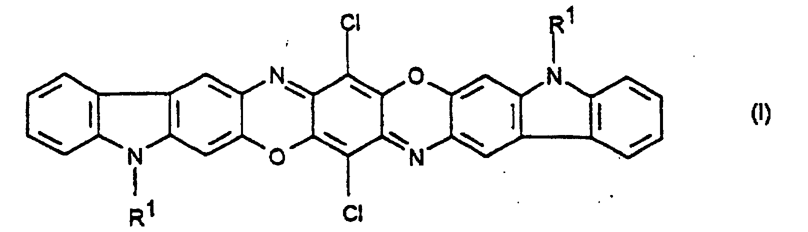 dans laquelle R¹ représente un atome d'hydrogène ou un groupe alkyle en C₁ à C₈ par cyclisation d'un composé de formule générale II 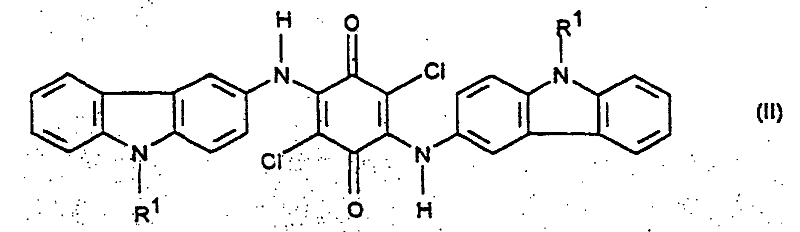 en présence d'un agent de cyclisation, **caractérisé en ce qu'**on utilise comme agent de cyclisation un N-hétarène-N-oxyde.

2. Procédé selon la revendication 1, **caractérisé en ce que**, R¹ représente le groupe éthyle.

3. Procédé selon la revendication 1 et/ou 2, **caractérisé en ce qu'**on utilise comme composés N-hétarène-N-oxyde des composés de formule générale III 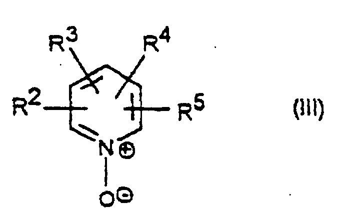 dans laquelle R², R³, R⁴ et R⁵ représentent indépendamment les uns des autres un atome d'hydrogène, un groupe alkyle en C₁ à C₄, un atome de chlore ou un groupe cyano, des composés des formules générales IV ou IVa 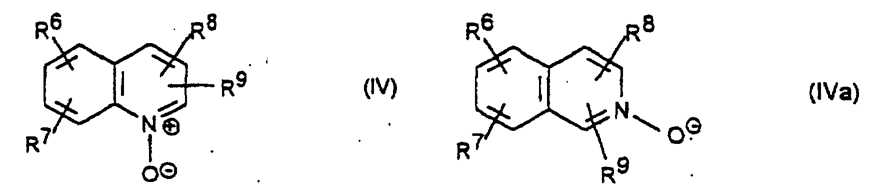 dans lesquelles R⁶, R⁷, R⁸ et R⁹ représentent indépendamment les uns des autres un atome d'hydrogène ou un groupe alkyle en C₁ à C₄,
ainsi que des composés de formule générale V 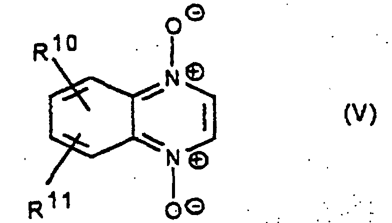 dans laquelle R¹⁰ et R¹¹ représentent indépendamment l'un de l'autre un atome d'hydrogène ou un groupe alkyle en C₁ à C₄.

4. Procédé selon une ou plusieurs des revendications 1 à 3, **caractérisé en ce qu'**on utilise comme N-hétarène-N-oxyde le pyridine-N-oxyde ou un picoline-N-oxyde.

5. Procédé selon une ou plusieurs des revendications 1 à 4, **caractérisé en ce qu'**on met en oeuvre le N-hétarène-N-oxyde en quantités de 1 à 3 moles, de manière particulièrement préférée en quantités de 1,2 à 2,5 moles, par mole de composé de formule générale II.

6. Procédé selon une ou plusieurs des revendications 1 à 5, **caractérisé en ce qu'**il est réalisé en présence d'un composé quinone.

7. Procédé selon la revendication 6, **caractérisé en ce qu'**on utilise comme composé quinone la p-benzoquinone ou la chloranile.

8. Procédé selon la revendication 6 et/ou 7, **caractérisé en ce qu'**on met en oeuvre le composé de quinone en quantités comprises entre 5 et 50 pour cent en mole, de manière particulièrement préférée entre 10 et 40 pour cent en mole, par rapport au composé de formule générale II.

9. Procédé de fabrication de composés de dioxazine de formule générale I 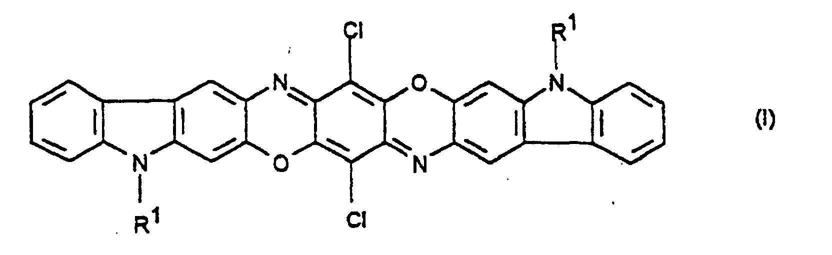 dans laquelle R¹ représente un atome d'hydrogène ou un groupe alkyle en C₁ à C₈ par condensation d'un 3-amino-N-alkyl-carbazol de formule générale VI 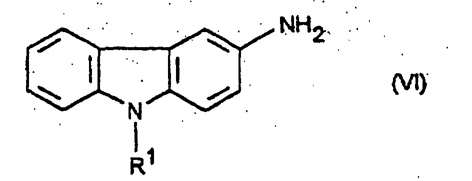 dans laquelle R¹ est défini comme indiqué ci-dessus, avec la chloranile dans un solvant organique inerte pour donner un produit de condensation de formule générale II 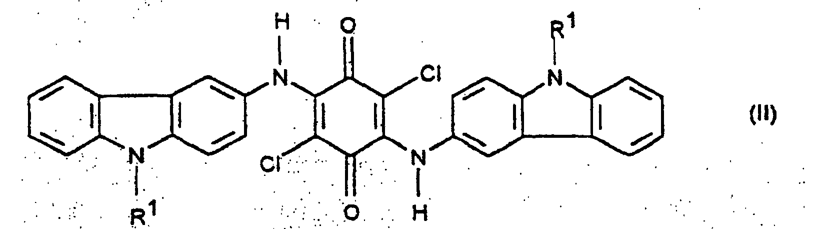 et ensuite cyclisation pour obtenir le composé de formule générale I en présence d'un agent de cyclisation, **caractérisé en ce qu'**on utilise comme agent de cyclisation un N-hétarène-N-oxyde.

10. Procédé selon la revendication 9, **caractérisé en ce que** le composé de formule générale II n'est pas isolé comme intermédiaire.
